# EUROPEAN PATENT APPLICATION

(11) **EP 4 141 028 A1**
(43) Date of publication of application: **01.03.2023**
(21) Application number: 21194170.3
(22) Date of filing: 31.08.2021
(51) Int. Cl.: C07K 16/28

(54) **PERSONALIZED VNAR-BASED CHIMERIC ANTIGEN RECEPTORS (VNAR-CAR) FOR THE TREATMENT OF CLONAL B-AND T CELL MALIGNANCIES, AND METHODS FOR PRODUCING VNAR-CARS**

(71) Applicant: Johann-Wolfgang-Goethe-Universität Frankfurt am Main, 60323 Frankfurt am Main (DE); Technische Universität Darmstadt, 64289 Darmstadt (DE)
(72) Inventor: Ullrich, Evelyn, 79110 Freiburg (DE); Wendel, Philipp, 64297 Darmstadt (DE); Wels, Winfried, 60599 Frankfurt am Main (DE); Oberoi, Pranav, 60528 Frankfurt am Main (DE); Kolmar, Harald, 74367 Mühltal (DE); Macarrón Palacios, Arturo, 64560 Riedstadt (DE); Habermann, Jan, 64297 Darmstadt (DE); Schoenfeld, Katrin, 55237 Bornheim (DE)
(74) Representative: Krauss, Jan

(57) **Abstract**

The present invention relates to a chimeric antigen receptor (CAR) molecule comprising at least one variable NAR (vNAR) antigen binding.domain. The variable NAR-domain specifically binds to a T cell or B cell specific antigen, and the variable NAR-domain binds to a specific idiotype of a B cell receptor (BCR) or T cell receptor (TCR) in a patient. The present invention further relates to uses of the CAR molecule in the treatment of clonal malignancies, such as T-ALL or B-ALL.

## Description

The present invention relates to a chimeric antigen receptor (CAR) molecule comprising at least one variable NAR (vNAR) antigen binding.domain. The variable NAR-domain specifically binds to a T cell or B cell specific antigen, and the variable NAR-domain binds to a specific idiotype of a B cell receptor (BCR) or T cell receptor (TCR) in a patient. The present invention further relates to uses of the CAR molecule in the treatment of clonal malignancies, such as T-ALL or B-ALL.

### Background of the invention

Acute lymphoblastic leukemia (ALL) is one of the most aggressive B cell non-Hodgkin malignancies known today. The malignancy affects children with a higher frequency (80%) compared to adults and is the most common pediatric malignancy. For children, a better initial treatment response can be observed, while treatment of adult ALL is characterized by a lesser long-term survival rate.

ALL is characterized by a malignant transformation of lymphoid progenitor cells residing in the blood, bone marrow and extramedullary sites, and can be classified by its distinct immunological phenotype into B cell- (~75%) or T cell ALL (~25%). Further subtypes of the B-ALL are characterized by the accompanied chromosomal abnormalities, such as chromosomal translocations, and mutations that result in an arresting differentiation and uncontrolled proliferation.

In general, the initial treatment strategy of B-ALL involves chemotherapy, followed by an allogeneic hematopoietic stem cell transplantation (HSCT) for high-risk patients. Development and optimization of various treatment concepts increased the poor treatment response of 10% in the 1960s to an initial response level of 90% for the adult and pediatric B-ALL patients today.

Unfortunately, treatment of relapsed or chemotherapy-refractory B-ALL is more challenging, and results in poor prognosis and survival rates for both adult and - even more important - childhood ALL. With a post relapse prognosis of 22% for 1-year and only 7% for 5-year survival, the further treatment remains challenging, and current approaches involve switching to other chemotherapeutics or application of targeted immunotherapy.

Although initial treatment of B-ALL with chemotherapy results in a high response and for most cases a progression free survival, treatment of refractory or relapsed B-ALL is still a hurdle for current therapeutic approaches.

During the last decades, immunotherapy evolved as a promising and highly effective therapy concept for treatment of various cancer types. With a focus on the treatment of B cell malignancies, several therapeutic antibodies were developed. These antibodies target common B cell surface proteins, such as CD20 or CD19. These strategies do not only have direct impact on the targeted malignant cells, but furthermore involve the naive capabilities of the immune system to effectively eliminate the malignant cell population.

As examples for antibody-based therapy approaches is the application of the anti-CD20 antibody rituximab or the T cell engager blinatumomab. The latter can be used as an alternative approach to chemotherapy and uses two scFv's binding the CD3 domain of T cells and the CD19 extracellular domain of B cells in order to provide a proximity of the effector cells towards target cells. This, in turn, promotes the formation of an immunological synapse and subsequent efficient elimination of the targeted B cell.

Other immunotherapeutic approaches for the treatment of B-ALL include a combination of immune checkpoint inhibitors (nivolumab) and therapeutic antibodies or immune cell products. A rapidly emerging immunotherapy approach is adoptive cell transfer (ACT): collecting and using patients' own immune cells to treat their cancer. There are several types of ACT ("TILs", "TCRs", and "CARs"), but, thus far, the one that has advanced the furthest in clinical development is called CAR-T-cell therapy, using autologous genetically modified immune cells, e.g. Chimeric antigen receptor (CAR)-T cell products (e.g., tisagenlecleucel, Kymriah^{®}). Like all cancer therapies, CAR T-cell therapy can cause several, and sometimes fatal, side effects. One of the most frequent is cytokine release syndrome (CRS).

Notably, immunotherapeutic strategies are highly favored for the treatment of B-ALL in patients with very high-risk of relapsed/refractory ALL. The applied treatment, nevertheless, heavily depends on the B-ALL subtype.

The mature B-ALL subtype Burkitt lymphoma or leukemia is the most common type of non-Hodgkin lymphoma (NHL) and accounts for 20% to 30% of pediatric lymphomas. It is characterized by an early involvement of the central nervous system (CNS) and a translocation of the *c-myc* proto-oncogene juxtaposed to the immunoglobulin heavy (IGH) or light chain (IGK or IGL) gene enhancers. Due to the important role of c-myc in the regulation of cell proliferation, this chromosomal translocation results in an unregulated proliferation of the malignant cells and promotes a fast doubling time of 24 to 48 hours.

Different clinical subtypes of Burkitt lymphoma are classified based as endemic, sporadic and immunodeficiency-associated and can be related to an exposure to Epstein-Barr virus (EBV).

As described for the B-ALL in general, Burkitt lymphomas are characterized by a high sensitivity towards chemotherapy treatment based on the fast proliferation rate. Thus, for Burkitt lymphoma a combination of classical therapy concepts, such as chemotherapy, and the application of therapeutic antibodies (e.g. rituximab) resulted in a higher efficacy of the treatment and a prolonged survival rate of the patient.

Contrary to other B-ALL subtypes, such as the pre-B-ALL, the Burkitt lymphoma cells are characterized as mature B cells, expressing common B cell surface markers like CD 19, CD20 and CD22 in addition to a clonal membrane bound IgM as B cell receptor. Due to the clonal expansion of the malignant population cells can be identified during evaluation of the minimal residual disease (MRD) though PCR or flow cytometry based on the unique clonal B cell receptor.

Considering the low 3-year survival rates of 18.5% for children with relapsed or refractory Burkitt lymphoma or Burkitt leukemia, there is an urgent need for the development of novel treatment strategies.

T-ALL is a subset of ALL and is a rare cancer: An estimated 6,150 new cases of ALL were diagnosed in 2020 in the United States; just 25% of these cases were in adults with T-ALL. Initial treatment is chemotherapy and allogeneic hematopoietic stem cell transplantation (HSCT). In contrast to B cell malignancies, it is not possible to deplete the T cells (e.g., anti-CD3 immunotherapy) since the loss of the T cell population would be fatal for the patient. A relapse or chemotherapy-refractory T-ALL is highly challenging with, very poor post relapse prognosis and no specific therapy available. Treatment options include further chemotherapy, radiation or (limited) immunotherapy. There are few therapeutic antibodies (Alemtuzumab, anti-CD52-antibody) that target not only T cells but also other leukocytes.

Current treatment approaches have severe side effects, such as B cell aplasia after CD 19-CAR therapy or application of therapeutic antibodies like blinatumomab. This highlights the urgent medical need for an effective and specific therapy, that can be tailored to the malignancy of individual patients for a personalized therapy concept.

It is therefore an object of the present invention to provide new and improved strategies for the immunotherapy of B-cell or T-cell related leukemias, that also avoid further disadvantages of the prior art. Other objects and advantages will become apparent to the person of skill when studying the present description of the present invention.

In a first aspect of the present invention, the above object is solved by a chimeric antigen receptor (CAR) molecule comprising at least one variable NAR-domain as antigen binding domain, wherein said variable NAR-domain specifically binds to a T cell or B cell specific antigen, and wherein said variable NAR-domain binds to a specific idiotype as defined by a B cell receptor (BCR) or T cell receptor (TCR) repertoire. Preferably, the CAR molecule according to the present invention is a CAR molecule of the first, second, third, fourth, fifth or next generation, optionally comprising one additional antigen binding domain, such as scFv antigen binding domain, VHH, DARPIN, or variable NAR-domain, and further optionally comprising at least one hinge region consisting of a flexible amino acid linker, such as (GGGGS)3-5 or a 6 to 12 glycine linker.

In a second aspect of the present invention, the above object is solved by a genetically modified or recombinant lymphocyte, such as a T cell, NK cell or other type of immune cell, expressing the CAR molecule according to the present invention, wherein preferably said lymphocyte, such as the T cell or NK cell, is derived from either autologous or third party donor (=allogeneic, haploidentical) origin.

In a third aspect of the present invention, the above object is solved by a method for producing the CAR molecule according to the present invention, in one embodiment comprising the steps of a) identifying the B cell receptor (BCR) and/or T cell receptor (TCR) idiotype(s) in a sample of B cells and/or T cells obtained from a patient, b) identifying at least one B cell receptor (BCR) and/or T cell receptor (TCR) idiotype specific variable NAR antigen binding domain comprising screening of a semi-synthetic library of potential variable NAR binding domains, and c) introducing the at least one variable NAR antigen binding domain as identified in step b) into a chimeric antigen receptor (CAR) molecule. In a second embodiment, the method for producing the CAR molecule according to the present invention comprises the steps of a) identifying at least one T cell or B cell idiotype specific variable NAR antigen binding domain comprising screening of a semi-synthetic library of potential variable NAR binding domains, b) including the at least one variable NAR antigen binding domain as identified in step a) into a chimeric antigen receptor (CAR) molecule, wherein said chimeric antigen receptor (CAR) molecule furthermore comprises at least one extracellular hinge region consisting of a flexible amino acid linker, such as (GGGGS)3-5 or a 6 to 12 glycine linker.

In a fourth aspect of the present invention, the above object is solved by a pharmaceutical composition, comprising the genetically modified, or recombinant lymphocyte, T cell or NK cell according to the present invention, or the vNAR-CAR-T cell or vNAR-CAR-NK cell as produced according to the present invention, together with pharmaceutically acceptable auxiliary agents, preferably a pharmaceutical composition that is suitable for adoptive cell therapy (ACT).

In a fifth aspect of the present invention, the above object is solved by the CAR molecule according to the present invention, the genetically modified, e.g. recombinant, lymphocyte, such as T cell or NK cell according to the present invention, the vNAR-CAR-T cell or vNAR-CAR-NK cell as produced according to the present invention, or the pharmaceutical composition according to the present invention for use in the treatment of diseases, such as clonal malignancies, such as leukemias, in particular ALL, such as T-ALL and B-ALL. Preferably, the treatment is personalized, in particular personalized immunotherapy.

As mentioned above, in a first aspect of the present invention, the above object is solved by a chimeric antigen receptor (CAR) molecule comprising at least one variable NAR-domain antigen binding domain, wherein said variable NAR-domain specifically binds to a T cell or B cell specific antigen, and wherein said variable NAR-domain binds to a B cell receptor (BCR) or cell T cell receptor (TCR) specific idiotype (repertoire). Preferably, the CAR molecule according to the present invention is a CAR molecule of the first, second, third, fourth or fifth or next generation, optionally comprising one additional scFv antigen binding domain or variable NAR-domain, and further optionally comprising at least one hinge region consisting of a flexible amino acid linker, such as (GGGGS)3-5 or a 6 to 12 glycine linker. In a second aspect of the present invention, the above object is then solved by a genetically modified, such as recombinant, lymphocyte, such as a T cell or NK cell, expressing the CAR molecule according to the present invention, wherein preferably said recombinant lymphocyte, such as the T cell or NK cell is an autologous T cell or NK cell, i.e. is derived from the same subject and/or idiotype, and is thus personalized.

An idiotype is a shared characteristic between a group of immunoglobulin or T-cell receptor (TCR) molecules based upon the antigen binding specificity and therefore structure of their variable region. The variable region of antigen receptors of T cells (TCRs) and B cells (immunoglobulins) contain complementarity-determining regions (CDRs) with unique amino acid sequences. They define the surface and properties of the variable region, determining the antigen specificity and therefore the idiotope of the molecule. Immunoglobulins or TCRs with a shared idiotope are the same idiotype.

In comparison to mammalian immunoglobulin isotypes, the evolution of other vertebrates gave rise to unique immunoglobulin isotypes that fulfill important niche functions in their adaptive immune system. One of those specialized immunoglobulins is the immunoglobulin new antigen receptor (IgNAR), that has been initially described in 1995 by Flajnik and colleagues after discovery in the blood of a nurse shark (Greenberg et al., 1995).

Contrary to immunoglobulin isotypes of humans, that form large hetero-tetrameric proteins, the IgNAR found in cartilaginous fish represents a heavy-chain only isotype with similar structure to the camelid heavy chain IgG (hcIgG). Antigen binding of the homodimer IgNAR is mediated by a single variable new antigen receptor (vNAR) domain connected to five constant domains (C1-C5). Thereby, the unique structure of IgNAR allows connection of the vNAR to the C1 domain in absence of a hinge region, while maintaining the property to bind multiple epitopes (Zielonka et al., 2015).

Compared to humans, vNARs have only the two complementarity determining regions CDR1 and CDR3. Instead of the CDR2 region found in variable domains of mammalian immunoglobulins, two hypervariable loops (HV2 and HV4) are present and influence the binding properties of the vNAR (Zielonka et al., 2015). The main diversity of the naive IgNAR repertoire is achieved through diversification of the CDR3 region of the vNAR. Notably, the elongated structure of the CDR3 binding loop enables a higher recognition diversity and enhances the binding potential towards cryptic and hidden epitopes, such as the active site of enzymes or clefts between two protein domains. Further information on structural insights of the vNAR domain can be found in Zielonka *et al.* (2015), incorporated herein by reference. In addition to the features provided by the elongate CDR3 loop, the small size of only 12 kDa as well as naive physicochemical stability and high solubility renders the vNAR an attractive protein scaffold for biomedical applications and novel therapy concepts. Furthermore, the enhanced ability to recognize the distinct paratope of immunoglobulins or Ig-based molecules can be utilized to select vNARs with nanomolar affinity towards B cell receptors on the surface of malignant B cells, in particular by screening of a semi-synthetic vNAR library derived from naive vNAR scaffolds of the bamboo shark (Könning et al., 2017; Macarrón Palacios et al., 2020; Zielonka et al., 2014, all incorporated herein by reference).

Most importantly, screening via yeast-surface display (YSD) resulted primarily in enrichment of anti-idiotype vNARs, able to recognize the distinct idiotype by interaction with the variable region of the targeted immunoglobulin (Könning et al., 2017, incorporated herein by reference). Thereby, the ability to quickly generate anti-idiotype vNARs against clonal immunoglobulin targets allowed the generation of a vNARs with high specificity towards the idiotype of the B-ALL cell line SUP-B8 as described in detail by Macarrón Palacios *et al.* (2020), also incorporated herein by reference.

EP2989203B1 relates to a synthetic library of antigen specific binding molecules derived from a member of a species in the Elasmobranchii subclass, processes for the production thereof and specific antigen specific binding molecules isolated from said library. The antigen specific antigen binding molecules have a peptide domain structure represented by the following formula (I): FW1-CDR1-FW2-HV2-FW3a-HV4-FW3b-CDR3-FW4.

WO2017196847A1 relates to the construction of a phage display variable new antigen receptor (VNAR) antibody library and the identification of antibody clones from the VNAR library that bind tumor and viral antigens, such as B-cell-epitopes.

EP3133085 discloses the generation of shark-based monoclonal antibodies biopharmaceuticals named vNAR that block the vascular endothelial growth factor (VEGF) and possess remarkable biological and biophysical properties.

EP3094354B1 discloses chimeric antigen receptors (CAR), the specificity of which is conferred by VNAR polypeptides derived from monomeric antibodies of cartilaginous fish. The CAR of the invention can be expressed at the surface of immune cells to redirect their specificity toward specific antigens. The CAR may further comprise a hinge region between its transmembrane region and its extracellular antigen recognition domain, and the entire extracellular domain may be shorter than 150 amino acids. The vNAR polypeptide sequence may be humanized.

EP3119423A2 relates to chimeric antigen receptors (CARs) specific to a cancer associated antigen as described herein, vectors encoding the same, and recombinant T cells comprising the CARs of the present invention. The application also discloses methods of administering a genetically modified T cell expressing a CAR that comprises an antigen binding domain that binds to a cancer associated antigen as described. The antigen binding domain may be connected to the transmembrane domain by a hinge region. In one embodiment, the encoded hinge region comprises the amino acid sequence of a CD8 hinge. Furthermore, flexible polypeptide linkers are mentioned in the context of scFvs that include (Gly4 Ser)4, (Gly4Ser)3 or multiple repeats of (Gly2Ser), (GlySer) or (Gly3Ser). vNAR is not mentioned.

As mentioned above, the present invention relates to a chimeric antigen receptor (CAR) molecule comprising a variable NAR, also called vNAR antigen binding domain. The variable NAR-domain preferably specifically binds to a T cell or B cell specific antigen as disclosed herein.

The CAR molecule according to the present invention has several features that provide advantageous effects. First, the vNARs, with a size of 11 to 12 kDa, are the smallest antigen-binding antibody-like domain in the animal kingdom, and thus offer steric advantages when replacing the commonly used scFv binder(s). The variable NAR-domain of the CAR then binds to a specific idiotype of a tumor cell B cell receptor (BCR) or tumor cell T cell receptor (TCR) repertoire.

T-ALL and B-ALL show a high clonality, i.e. the population shares a tumor-specific idiotype with a unique set of T cell receptor (TCR) α/β or TCR γ/δ or BCR heavy/light chain. This clonality allows for targeting of the tumor-specific idiotype, while sparing the healthy immune cell population, thus assuring a high selectivity for the tumor cells and no or limited off-target effects for the healthy population. In case of many B-cell lymphomas, the tumor cells express a tumor-specific and functionally active BCR, also known as idiotype. Utilizing the idiotype as target for lymphoma therapy has emerged to be demanding since the idiotype differs from patient to patient. Previous studies have shown that shark-derived antibody domains (vNARs) isolated from a semi-synthetic CDR3-randomized library allow for the rapid generation of idiotype-related binders.

Macarrón Palacios et al., 2020 disclose methods of generating patient-specific binders against the idiotype of lymphomas.

In contrast to the described limits of current CAR-T cell therapies, the exclusive recognition of the unique tumor-idiotype by the anti-idiotype-CAR-T/-NK cell therapy does not only allow a widening of the therapeutic window for patients with tumor relapse or refractory status, but furthermore a maintenance of the healthy immune cell population of the patient, preventing severe side effects.

The CAR molecule according to the present invention further optionally comprises at least one extracellular hinge region consisting of a flexible amino acid linker or spacer, such as (GGGGS)3-5 or a 6 to 12 glycine linker. Preferably, this amino acid linker or spacer replaces the CD8α hinge domain, preferably with a glycine-serine-linker (G4S) with four repeats, resulting in the vNAR-CAR with the shortest extracellular domain.

The exchange of a commonly used scFv towards vNARs as novel binding element and targeting of the SUP-B8 BCR required an adaptation of extracellular spacer domains to provide correct signal transduction and high flexibility. In comparison to common targets for immunotherapy of B-ALL, such as the extracellular domain of CD19, the B cell receptor represents a significantly larger heterotetrametric protein complex on the surface membrane of B cells. Considering size and distance differences of the targeted epitope towards the vNAR-CAR, a screening of various extracellular spacer regions domains was required to select the optimal spacer domain for the vNAR-CAR to promote optimal binding properties. Five extracellular domains were designed *in silico,* characterized by different spacer/hinge modifications and relocation of the myc-tag, see examples below. Preferred is the CAR molecule according to the present invention comprising a short and flexible hinge region based on a (G₄S)₄-linker replacing or essentially replacing (e.g. except for a transmembrane sequence of CD8α), e.g., the commonly implemented CD8α hinge domain (see below for a description of common structures of CAR).

Preferably, the CAR molecule according to the present invention is a CAR molecule of the first, second, third, fourth or fifth or next generation, optionally comprising one additional antigen binding domain, such as an scFv antigen binding domain, VHH, DARPIN, or variable NAR-domain.

A typical CAR has an extracellular single-chain variable fragment (scFv), connected through a hinge/spacer domain and transmembrane domain to intracellular (costimulatory) signaling domains. The recognition of tumor antigens by scFv activates the intracellular costimulatory signaling domain, subsequently stimulating T cell proliferation and eliminating tumor cells through cytolysis and cytokine release. Based on the structure of the intracellular region, CARs are divided into at least five generations. First-generation CARs contain only one intracellular signaling domain, CD3ζ. In contrast, second- and third-generation CARs utilize first-generation CARs as a backbone and incorporate one or two additional costimulatory signaling domains, such as CD28 or 4-1BB (CD137), respectively. The fourth-generation CARs, based on the second-generation CARs, can induce cytokine production. The fifth-generation CARs are also based on the second-generation CARs, containing intracellular domains of cytokine receptors, such as IL-2Rβ chain fragment. The exemplary CAR according to the present invention are based on second-generation CARs. Unlike T cell receptors (TCRs), CARs recognize specific surface targets on tumor cells in a major histocompatibility complex (MHC)-unrestricted manner, effectively preventing immune escape by downregulating MHC expression in tumor cells.

Preferred is a CAR molecule according to the present invention, wherein the vNAR binds or specifically binds to a (at least one) B or T cell specific antigen, more preferably a mammalian, such as a human antigen. The invention is not limited to a specific antigen, preferably the antigen is selected from the group consisting of survivin, CD19, CD20, CD22 and CD52 for B-ALL, or CD34, CD117, CD13, and CD33 for T-ALL, such as CD1a, CD2, CD3, CD4, CD5, CD13, CD19, CD20, CD22, CD30, CD33, CD34, CD44s, CD52, CD99, CD123, IL7R, CXCR4, survivin, CD138, BCR, and TCR.

An exemplary and particularly preferred embodiment CAR molecule according to the present invention has an amino acid sequence according to SEQ ID NO: 1, or sequences being at least 95%, preferably at least 97% and most preferably at least 99% identical thereto, wherein the vNAR is preferably according to SEQ ID NO: 3, or sequences being at least 95%, preferably at least 97% and most preferably at least 99% identical thereto. This CAR molecule includes a short and flexible hinge region based on a (G4S)4-linker, essentially replacing (except for a transmembrane sequence of CD8α) the CD8α hinge domain.

Yet another aspect of the present invention then relates to a nucleic acid, encoding for the CAR molecule according to the present invention, such as a DNA or RNA, wherein said nucleic acid preferably is part of an expression cassette or vector, such as a plasmid or viral vector, e.g. a lentiviral vector or transposon-based system. Respective expression cassettes or vectors are also known to the person skilled in the art.

Yet another aspect of the present invention then relates to a recombinant leukocyte, T cell or NK cell, expressing the CAR molecule according to the present invention, wherein preferably said T cell or NK cell is an autologous T cell or NK cell for a specific subject or patient, such as a cancer patient, in particular a T-ALL or B-ALL or refractory T-ALL or B-ALL patient. Patients can further be selected from pediatric or adult patients.

T-ALL and B-ALL show a high clonality, i.e. the population shares a tumor-specific idiotype with a unique set of T cell receptor (TCR) α/β or BCR heavy/light chain. This clonality allows for an essentially selective targeting of the tumor-specific idiotype while sparing the healthy immune cell population, thus assuring a high selectivity for the tumor cells and no or limited off-target effects for the healthy cell population. Thus, the present invention allows for a next-generation personalized immunotherapy that combines the clinically proven potential of CAR-based immunotherapy with novel functional elements (vNARs and short and flexible linkers as above) that allow the improved recognition of clonal antigen receptor idiotypes as a patient-specific antigen pattern unique to the malignant population. With the recent focus on clinical NK cell-based therapies, the invention also includes CAR-NK cell engineering and combining the advantages of the effective naive cytotoxic capability of NK cells with the low risk of causing T cell-related side effects in an allogeneic setting, such as Graft-vs-Host-Disease (GvHD) and cytokine release syndrome (CRS).

Yet another aspect of the present invention then relates to a method for producing the CAR molecule according to the present invention, comprising the steps of a) identifying at least one anti-idiotype specific variable NAR B or T cell specific antigen binding domain comprising screening of a semi-synthetic library of potential variable NAR binding domains, b) including the at least one anti-idiotype specific variable NAR B or T cell specific antigen binding domain as identified in step a) into a chimeric antigen receptor (CAR) molecule, replacing at least one scFv antigen binding domain by said variable NAR-domain, wherein said chimeric antigen receptor (CAR) molecule furthermore comprises at least one extracellular hinge region consisting of a flexible amino acid linker, such as (GGGGS)3-5 or a 6 to 12 glycine linker, preferably replacing or essentially replacing (e.g. except for a transmembrane sequence of CD8), e.g., the commonly implemented CD8α costimulatory signaling domain.

Anti-idiotype screening with a semi-synthetic vNAR library (Könning et al., 2017) results primarily in vNARs that are able to recognize the specific paratope of immunoglobulins, such as the B-cell receptor.

Preferred is the method according to the present invention, further comprising the step of humanizing the variable NAR-domain as identified. Methods to humanize CAR molecules are known in the art, see, for example, EP3094354B1 as cited above.

As mentioned above, the method according to the present invention may be used to produce the CAR molecule according to the invention, wherein the CAR molecule is of the first, second, third, fourth or fifth generation, optionally comprising one additional scFv antigen binding domain or variable NAR-domain.

Another aspect of the present invention then relates to the method according to the present invention, further comprising the step of expressing said CAR molecule in a host cell, such as a vNAR-CAR-T cell or vNAR-CAR-NK cell, preferably in an autologous T cell or NK cell. Preferably the T cell or NK cell is an autologous T cell or NK cell for a specific subject or patient, such as a cancer patient, in particular a T-ALL or B-ALL or refractory T-ALL or B-ALL patient. Patients can further be selected from pediatric or adult patients. Methods for transfecting/transducing and expressing the nucleic acids encoding the CAR molecule in a host cell are known in the art, see, for example, below for vNAR-CAR expressing Jurkat cells (CD3+/eGFP+).

Generated vNAR candidates are screened to prevent off-target effects and confirm selectivity for specific idiotype, preferably by monitoring of specific and non-specific binding-ratio by labeling of heterogenous population consisting of target cells with PBMC (see below).

The cells as produced may then be used, amongst others, to generate a pharmaceutical composition for treating a cancer patient, in particular a T-ALL or B-ALL or refractory T-ALL or B-ALL patient. For the development of the composition and/or other properties of the composition, the method according to the present invention, may further comprise the step of detecting the antigen target-specificity, off-target side effect, and/or vNAR-CAR-mediated cytotoxicity of said vNAR-CAR-T cell or vNAR-CAR-NK cell against a patient-derived tumor sample, and/or identifying the clonal/antigen-specific idiotype of the B cell receptor (BCR) and/or T cell receptor (TCR) repertoire in a sample of B cells and/or T cells obtained from a patient.

Yet another aspect of the present invention then relates to a pharmaceutical composition, comprising the CAR molecule according to the present invention, the recombinant leukocyte, T cell or NK cell according to the present invention, or the vNAR-CAR-T cell or vNAR-CAR-NK cell as produced according to the present invention, together with pharmaceutically acceptable auxiliary agents, preferably a pharmaceutical composition that is suitable for autologous cell therapy (ACT). Particular preferred is a personalized therapy based on the clonal idiotype of the disease to be treated, e.g. leukemia, such as T-ALL or B-ALL. A pharmaceutical composition according to the present invention preferably are anti-idiotype vNAR-CAR-engineered lymphocytes, including lymphocyte-subsets and cell lines, as immunotherapeutic product. Such formulations of therapeutic agents as a pharmaceutical composition may be prepared for storage by mixing with physiologically acceptable carriers, excipients, or stabilizers in the form of, e.g., lyophilized powders, slurries, aqueous solutions or suspensions (see, e.g., Hardman, et al. (2001) Goodman and Gilman's The Pharmacological Basis of Therapeutics, McGraw- Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

Another aspect of the present invention then relates to the CAR molecule according to the present invention, the recombinant leukocyte, T cell or NK cell according to the present invention, the vNAR-CAR-T cell or vNAR-CAR-NK cell as produced according to the present invention, or the pharmaceutical composition according to the present invention for use in the treatment of diseases. Preferred is the CAR molecule according to the present invention, the recombinant leukocyte, T cell or NK cell according to the present invention, the vNAR-CAR-T cell or vNAR-CAR-NK cell as produced according to the present invention, or the pharmaceutical composition according to the present invention for use according to the present invention, wherein said disease is selected from clonal malignancies, such as leukemias, in particular ALL, such as T-ALL and B-ALL. More preferably, said treatment is personalized, in particular personalized immunotherapy, i.e. tailored to the malignancy of individual patients for a personalized therapy concept.

Another aspect of the present invention then relates to a method for treating clonal malignancies, such as leukemias, in particular ALL, such as T-ALL or B-ALL, comprising administering to a patient in need of said treatment an effective amount of the CAR molecule according to the present invention, the recombinant leukocyte, T cell or NK cell according to the present invention, the vNAR-CAR-T cell or vNAR-CAR-NK cell as produced according to the present invention, or the pharmaceutical composition according to the present invention, wherein preferably said treatment is personalized, in particular personalized immunotherapy, i.e. tailored to the malignancy of individual patients for a personalized therapy concept.

The term "subject" as used herein refers to a human or non-human organism. Thus, the methods and compositions described herein are applicable to both human and veterinary disease. In certain embodiments, subjects are "patients," i.e., living humans that are receiving medical care for a disease or condition. This includes persons with no defined illness who are being investigated for signs of pathology. "Therapeutically effective amount" is defined as an amount of a reagent or pharmaceutical composition that is sufficient to induce a desired immune response specific for encoded heterologous antigens, show a patient benefit, i.e., to cause a decrease, prevention, or amelioration of the symptoms of the condition being treated.

The cell compositions described herein can be administered to a host, either alone or in combination with a pharmaceutically acceptable excipient, in an amount sufficient to induce an appropriate anti-tumor response. The response can comprise, without limitation, specific immune response, non-specific immune response, both specific and non-specific response, innate response, primary immune response, adaptive immunity, secondary immune response, memory immune response, immune cell activation, immune cell proliferation, immune cell differentiation, and cytokine expression.

It can generally be stated that a pharmaceutical composition comprising the CAR-T cells described herein may be administered at a dosage of 10⁴ to 10¹¹ cells/kg body weight, preferably 10⁷ to 10¹⁰ cells/kg body weight, including all integer values within those ranges. T cell compositions may also be administered multiple times at these dosages. The cells can be administered by using infusion techniques that are commonly known in immunotherapy (see, e.g., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

Current therapy approach for treatment of refractory or relapse B-ALL and T-ALL are limited and may include severe side effects. This highlights the urgent medical need for the development of novel therapy approaches. With the aim to combine the anti-idiotype binding properties of the vNAR and the excellent efficacy of CAR-based immunotherapies, the present invention provides the proof-of-concept for an anti-idiotype vNAR-CAR therapy approach.

Initial steps during the proof-of-concept included the structure-guided *in silico* design for adaptation of extracellular vNAR-CAR domains. Thereby, glycine-serine-linkers of different length were implemented as space domains to promote optimal flexibility. Under consideration, that a further shortening of the extracellular domain promotes an exclusion of large inhibitory receptors interfering with CAR-signal transduction, the design of vNAR-CAR (5) was characterized by the shortest and most flexible hinge region based on a (G4S)4-linker replacing the commonly implemented CD8α domain. After successful *in silico* design of the extracellular vNAR-CAR domains, the pSIEW-vNAR-CAR lentiviral transfer plasmids were generated in a 2- or 3-step cloning procedure for the application in nucleofection and production of lentiviral particles. Simultaneously, an optimized nucleofection protocol was established, enabling a transient expression of the generated vNAR-CAR via transfection of the T-ALL cell line Jurkat. Subsequent screening of vNAR-CAR (1)-(5) using nucleofection- and transduction-based CD69 T cell activation assays (CD69 assay) demonstrated highly promising results for vNAR-CAR (5). This construct revealed superior results in terms of achieved transduction efficiency, specificity towards the target cell line SUP-B8, signal intensity and minimal off-target effects even against a heterogenous population of primary B cells. Subsequent analysis additionally demonstrated a vNAR-mediated cytotoxic effect on SUP-B8 cells indicated by significant decrease of the SUP-B8 population after co-culture with vNAR-CAR (5) modified Jurkat cells.

With conclusion of the initial proof-of-concept, this work highlights the vNAR-CAR as a novel tool for specific recognition of idiotypes found in clonal malignancies. The promising results of vNAR-CAR (5) not only prove the feasibility of the concept but furthermore promote the transfer of the vNAR-CAR system into a primary NK cell-based setting to evaluate of the combined vNAR-CAR-mediated and naive cytotoxic potential of NK cells *in vitro* and *in vivo.* Furthermore, generation of specific vNARs against the idiotype of the Burkitt lymphoma cell line Ramos is anticipated, due to the highly similar BCR isotype (IgM, lambda light chain). In addition, the vNAR-based CAR concept may also allow to generate idiotype-specific CARs directed against other Ig-like targets such as the clonal T-cell receptors expressed by T-ALL cells and other T-cell malignancies.

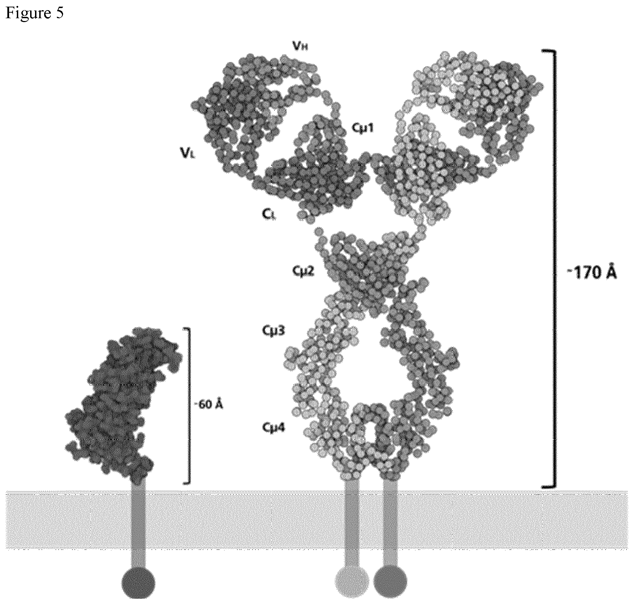

With a focus on enhanced specificity and reduced off-target effects for B-ALL, this work highlights the vNAR-CARs as a novel tool for the specific recognition of idiotypes found in clonal malignancies, such as the Burkitt lymphoma. Considering the clonal expansion of the malignant population, the tumor cells not only share common B cell antigens found on healthy B cells, such as CD 19, but are identifiably by their unique and clonal idiotype. This emphasize specific targeting of the clonal idiotype, to spare the healthy B cell population. This work describes the development of a vNAR-CAR against the Burkitt lymphoma cell line SUP-B8 with the objective to establish a proof-of-concept for the feasibility and efficacy of a vNAR-based therapy approach. Initial structure-based design was defined by analysis and comparison of available protein structures of the B cell receptor and the extracellular domain of CD 19. The increased size of the targeted B cell receptor required an adaptation of the CAR spacer region to promote optimal binding properties for the implemented vNAR (Figure 5). Five extracellular domains were designed *in silico,* characterized by different spacer/hinge modifications and relocation of the myc-tag. Cloning of the extracellular domains, containing the vNAR, myc-tag, glycine-serine-linker with various repeats and/or the CD8α hinge region, into the pMK intermediate plasmid resulted in the assembly of the full-length CAR gene. Subsequent cloning was performed to implement the vNAR-CAR into the pSIEW lentiviral transfer vector. Analysis of the different spacer modification was conducted with an optimized CD69 T cell activation assay (CD69 assay) as high-throughput technique for characterization of novel CAR constructs. This CD69 assay is based on the transient genetic modification of the T-ALL cell line Jurkat through nucleofection for expression of the desired CAR construct, followed by evaluation of the activation marker CD69 as readout for functional CAR signal transduction. After establishment of an optimized Jurkat nucleofection protocol, vNAR-CARs (1)-(5) were evaluated in a nucleofection-based CD69 assay with focus on specificity towards the target cell line SUP-B8 and activation potential. As anticipated during *in silico* design, the vNAR-CAR with the shortest extracellular domain, vNAR-CAR (5), revealed highly promising results, characterized by a high and specific expression of CD69 after coculture with SUP-B8, indicating a functional vNAR-CAR. The further experimental procedure involved the production of lentiviral particles for a stable transduction of Jurkat cells with the generated vNAR-CARs. Analysis of the specificity and activation potential via CD69 assay highlighted vNAR-CAR (5) again as a lead candidate, with minimal off-target effects against the pre-B-ALL line SUP-B15, the cell line IM-9 and a heterogenous population of primary B cells. Preliminary evaluation of a vNAR-CAR-mediated cytotoxic effect on SUP-B8 cells revealed significant decrease of the SUP-B8 population after co-culture with vNAR-CAR (5) modified Jurkat cells. The promising results achieved during the work promote a further analysis of the vNAR-CAR (5) specificity in a vNAR-CAR-NK cell setting and evaluation of the combined naive and vNAR-CAR-mediated cytotoxic potential of NK cells. Subsequent procedure involves the extension of the proof-of-concept towards *in vivo* experiments in direct comparison with current CD19-CAR therapy approaches.

The present invention will now be described further in the following examples with reference to the accompanying Figures and Sequence listing, nevertheless, without being limited thereto. For the purposes of the present invention, all references as cited herein are incorporated by reference in their entireties. In the Figures,

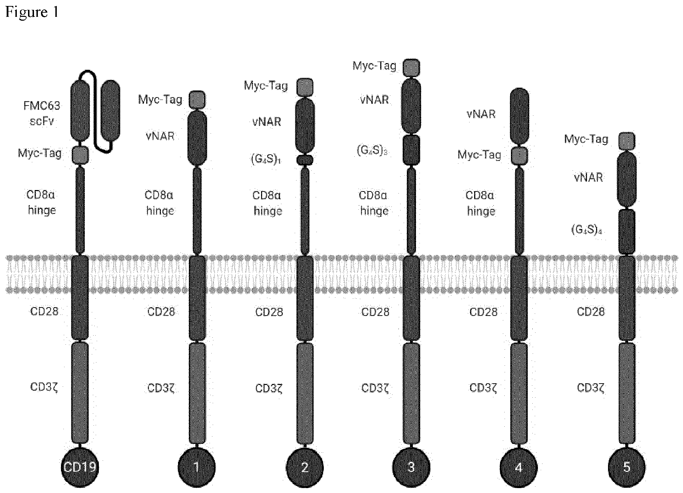

Figure 1 shows a schematic illustration of membrane-bound CD19-CAR and vNAR-CARs as designed in the context of the invention. Shown are schematic representations of vNAR-CAR domains in comparison to the CD19-CAR (CD19) in a membrane-bound state. The membrane is illustrated as phospholipid bilayer. The size of each domain is only estimated and does not necessary reflect actual domain size. Notably, all screened constructs contained a CD8α hinge domain with the exception of vNAR-CAR (5). Here, the CD8α hinge domain was replaced with a glycine-serine-linker (G₄S) with four repeats, resulting in the vNAR-CAR with the shortest extracellular domain. The following abbreviations are indicated: scFv = single chain variable Fragment; Myc-Tag = c-myc-protein tag; CD8α hinge = hinge region of human CD8α chain; CD28 = transmembrane and signaling domain of human CD28; CD3ζ= Human CD3ζ chain signaling domain; vNAR = variable domain of IgNAR; (G₄S)_{X}= Glycine-serine-linker with X repeats.

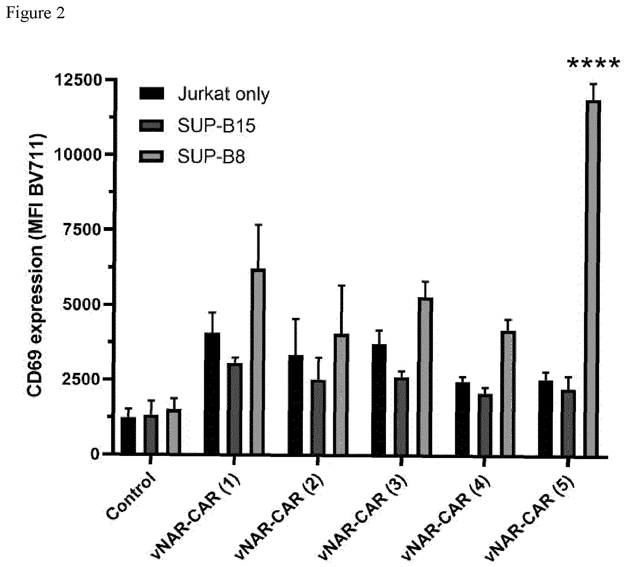

Figure 2 shows the nucleofection-based screening of vNAR-CARs in CD69 T cell activation assays. Jurkat cells were nucleofected with pulse setting CA-138 and 5µg lentiviral transfer plasmid of vNAR-CAR (1)-(5) or in absence of plasmid DNA (control). After regeneration for 24 hours at 37°C, nucleofected cells were co-cultured with SUP-B15 (off-target) or SUP-B8 cells (target) for 23-25 hours (E:T = 2:1). For each condition, Jurkat cells were cultured without target cells as control (Jurkat only control). Cells were stained with anti-CD3 BUV395, anti-CD69 BV711 and anti-c-myc PE to evaluate to upregulation of CD69 after co-culture via flow cytometry. Shown are median fluorescence intensities (MFI) of the CD69 BV711 channel of vNAR-CAR expressing Jurkat cells (CD3+/eGFP+) and control Jurkat cells (CD3+/eGFP-). Indicated asterisk [∗∗∗∗] represents a significant difference (p < 0.0001) against all other SUP-B8 conditions (one-way ANOVA). N (experiments) = 2; n (replicates) = 2; Error bars = +/-SD

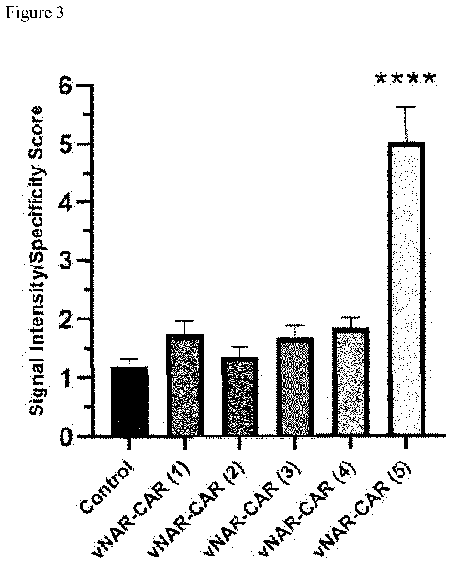

Figure 3 shows vNAR-CAR signal intensity and specificity after nucleofection-based screening. Compared are vNAR-CAR (1)-(5) using the vNAR-CAR Signal Intensity/Specificity Score, to determine specific activation of each vNAR-CAR against the SUP-B8 cell line (E:T = 2:1). The score was calculated based on median fluorescence intensity (MFI) of the CD69 (channel BV711) after 24-hour co-culture during the CD69 T cell activation assay. Indicated asterisk [****] represents a significant difference (p < 0.0001) against all other Signal Intensity/Specificity scores (one-way ANOVA). N (experiments) = 2; n (replicates) = 2; Error bars = +/-SD

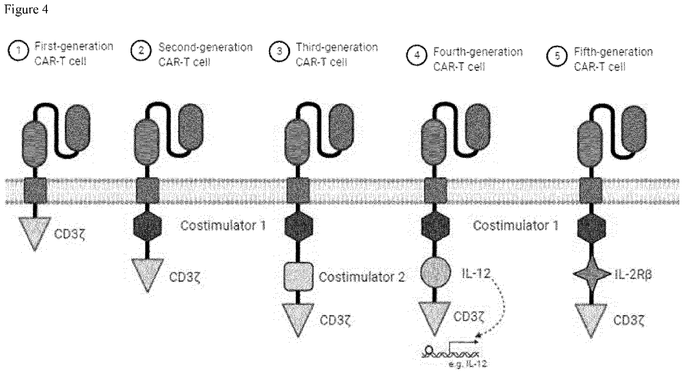

Figure 4 shows a schematic illustration of the structures of common CARs.
Figure 5 shows a schematic illustration of CD19 and BCR. In silico design of vNAR-CARs required optimization of extracellular CAR domains to adapt the vNAR-CAR system to the increased size of the targeted B cell receptor (surface membrane-bound IgM of SUP-B8) in comparison to CD19. Shown are representative illustrations of the following extracellular protein domains: CD19 extracellular domain (blue) based on pdb entry [6AL5]. Monomeric IgM heavy (green) and light chains (violet) and corresponding subdomains as surrogate for the SUP-B8 B cell receptor (BCR) based on pdb entry [2RCJ]. The sizes of each depicted protein was estimated. The increased size of the targeted B cell receptor required an adaptation of the CAR spacer region to promote optimal binding properties for the implemented vNAR.

### Examples

The experiments as performed in the context of the present invention confirmed that by selecting patient-specific vNAR-CAR-constructs and the screening for the specific idiotype of the malign cell population a high selectivity for tumor cells and low off-target-effects are triggered in the healthy population of cells. The lead-candidate construct vNAR-CAR (5) showed a high specificity and minimal off-target effects against the Burkitt-Lymphoma-cell line SUP-B8. Furthermore, the tumor subtype-specific cytotoxicity of the vNAR-CAR-constructs was confirmed against the SUP-B8-lymphoma cells, while no cytotoxicity was observed against control cells and a different lymphoma cell line, SUP-B15.

### Production of vNAR-CAR

The following vNAR-CAR constructs were designed.

| **Construct** | **Details** | **Size in base pairs (bp)** |
|---|---|---|
| **vNAR-CAR (1)** | SP-Myc-vNAR-CD8α | 633 |
| **vNAR-CAR (2)** | SP-Myc-vNAR-(G₄S)₁-CD8α | 648 |
| **vNAR-CAR (3)** | SP-Myc-vNAR-(G₄S)₃-CD8α | 678 |
| **vNAR-CAR (4)** | SP-vNAR-Myc-CD8α | 633 |
| **vNAR-CAR (5)** | SP-Myc-vNAR-(G₄S)₄ | 528 |

The DNA fragments were designed in silico and were codon-optimized (human) prior to synthesis. With exception of vNAR-CAR (5), all vNAR-CARs possess a CD8α hinge domain. The following abbreviations were used: SP = Signal peptide of the IgG heavy chain; Myc = c-myc protein tag; aSUP-B8 vNAR = vNAR with high affinity towards the BCR (idiotype) of the cell line SUP-B8; (G₄S)_{X} = Glycine-serine linker with various repeats of [GGGGS] (SEQ ID NO: 5); CD8alpha = CD8alpha hinge/transmembrane domain.

The following listing shows the sequences for the CAR construct vNAR-CAR (5) according to the present invention, as well as for the vNAR-domain as used.

SEQ ID NO: 1; vNAR-CAR (5); amino acid sequence

MDWIWRILFLVGAATGAHSEQKLISEEDLMAARLEQTPTTTTKEAGESLTINCVLKGSK
YALGNTYWYFTKKGATKKASLSTGGRYSDTKNTASKSFSLRISDLRVEDSGTYHCEAYF

AGFHYMLKRHIEGGGTTLTVKGGGGSGGGGSGGGGSGGGGSLQTRGLDKPFWVLVVVGG
VLACYSLLVTVAFIIFWVRSKRSRLLHSDYMNMTPRRPGPTRKHYQPYAPPRDFAAYRS
RVKFSRSADAPAYQQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPPR (SEQ
ID NO: 1)

The construct consists of: Signal peptide for Ig heavy chain - Myc tag (underlined) - Anti-SUP-B8-BCR vNAR clone F2 - glycine-serine-linker (bold) - minimal CD8 hinge membrane anchor (double underlined) - CD28 costimulatory domain - CD3 zeta domain (italic).

SEQ ID NO: 2; vNAR-CAR (5); DNA sequence

ATGGACTGGATTTGGAGAATCCTGTTCCTCGTGGGAGCCGCCACAGGCGCTCATTCTGA
GCAGAAGCTGATCTCCGAAGAGGACCTGATGGCTGCTAGACTGGAACAGACCCCTACCA
CCACCACAAAAGAGGCCGGCGAGAGCCTGACCATCAACTGTGTGCTGAAGGGCTCTAAG
TACGCCCTGGGCAACACCTACTGGTACTTCACCAAGAAGGGCGCCACAAAGAAGGCCAG
CCTGTCTACAGGCGGCAGATACAGCGACACCAAGAACACCGCCAGCAAGAGCTTCAGCC
TGCGGATCAGCGATCTGAGAGTGGAAGATAGCGGCACCTACCACTGCGAGGCCTACTTT
GCCGGCTTCCACTACATGCTGAAGCGGCACATTGAAGGCGGCGGAACCACACTGACCGT
GAAAGGCGGAGGCGGAAGTGGAGGCGGAGGAAGCGGAGGGGGAGGATCAGGCGGCGGAG
GGTCCCTGCAGACCAGGGGCCTGGACAAGCCCTTCTGGGTGCTGGTCGTGGTCGGCGGC
GTGCTGGCCTGTTACAGCCTGCTGGTCACCGTGGCCTTCATCATCTTTTGGGTCCGCAG
CAAGAGAAGCCGGCTGCTGCACTCCGACTACATGAACATGACCCCCAGACGGCCTGGCC
CCACCAGAAAGCACTACCAGCCCTACGCCCCTCCTAGAGATTTCGCCGCCTACCGGTCC
AGAGTGAAGTTCAGCAGATCCGCCGACGCCCCTGCCTATCAGCAGGGCCAGAACCAGCT
GTACAACGAGCTGAACCTGGGCAGACGGGAGGAATACGACGTGCTGGACAAGAGAAGAG
GCCGGGACCCTGAGATGGGCGGCAAGCCCAGACGGAAGAACCCCCAGGAAGGCCTGTAT
AACGAACTGCAGAAAGACAAGATGGCCGAGGCCTACAGCGAGATCGGCATGAAGGGCGA
GCGGAGAAGAGGCAAGGGCCACGACGGCCTGTACCAGGGCCTGAGCACCGCCACCAAGG
ACACCTACGACGCCCTGCACATGCAGGCTCTGCCACCTAGATAA

SEQ ID NO: 3; vNAR of construct 5; amino acid sequence

MAARLEQTPTTTTKEAGESLTINCVLKGSKYALGNTYWYFTKKGATKKASLSTGGRYSD
TKNTASKSFSLRISDLRVEDSGTYHCEAYFAGFHYMLKRHIEGGGTTLTVK

SEQ ID NO: 4; vNAR of construct 5; DNA sequence

ATGGCTGCTAGACTGGAACAGACCCCTACCACCACAAAAGAGGCCGGCGAGAGCCT
GACCATCAACTGTGTGCTGAAGGGCTCTAAGTACGCCCTGGGCAACACCTACTGGTACT
TCACCAAGAAGGGCGCCACAAAGAAGGCCAGCCTGTCTACAGGCGGCAGATACAGCGAC
ACCAAGAACACCGCCAGCAAGAGCTTCAGCCTGCGGATCAGCGATCTGAGAGTGGAAGA
TAGCGGCACCTACCACTGCGAGGCCTACTTTGCCGGCTTCCACTACATGCTGAAGCGGC
ACATTGAAGGCGGCGGAACCACACTGACCGTGAAA

### CD69 T cell activation assay

Published by Bloemberg *et al.* in 2020, the CD69 T cell activation assay was used as a high-throughput screening technique for characterization of novel CAR constructs with a focus on off-target effects and functional signal transduction (Bloemberg et al., 2020). In brief, Jurkat cells are genetically modified to obtain a transient expression of the desired CAR constructs. During a following co-culture with antigen-carrying target cells, modified Jurkat cells are activated through CAR-antigen-interaction and subsequent signal transduction via the CD3ζ domain of the CAR.

As described for its naive function in the TCR, the signaling through the CD3ζ domain leads among others to an upregulation of CD69 (Bloemberg et al., 2020). This upregulation was monitored via immunofluorescent staining of CD69 and following flow cytometry analysis. Detailed evaluation of the CD69 expression level after co-culture with target- and off-target cells allowed a prediction of the CARs specificity as well as signal strength after antigen contact. Ideally, the examined CAR mediates only a small activation for off-target cell lines, which might occur due to naive receptor-ligand interaction, and a strong activation exclusively for the antigen-carrying target cell line.

For the experiments, the protocol of Bloemberg and colleagues was slightly adapted to the established and optimized nucleofection protocol for Jurkat cells. Further modifications were made by extension of the regeneration time after nucleofection from 4 to 24 hours, in favor of an increased viability, and the utilized staining of the cells. In detail, Jurkat cells were nucleofected with the lentiviral transfer plasmids carrying vNAR-CAR (1)-(5) or CD19-CAR. After a regeneration period of 24h hours, 4 × 10⁴ viable nucleofected Jurkat cell were seeded into a 96-well plate (flat bottom), followed by addition of 2 × 10⁴ target/off-target cells for a total volume of 200µL RPMI 1640 growth medium per well. The utilized effector to target cell ratio of 2:1 was selected based on the higher doubling time of the SUP-B8 target cells in comparison to the Jurkat cells and resulting overgrowth at E:T of 1:1. Nucleofected Jurkat cells seeded with 100µL medium instead of target cells served as additional control and has been termed *Jurkat only control* for the following description. Among others, this control allowed a determination of constitutive CAR activation (*tonic signaling*)*.* As a control for the assay, unmodified Jurkat cells were treated with the protein kinase C activator phorbol 12-myristate 13-acetate (PMA, 50 ng/ml) in combination with Ionomycine (500 ng/ml) for 24 hours, to induce an unnatural high upregulation of CD69.

After co-culture for 24 hours, the cells were resuspended and stained with anti-c-myc PE, anti-CD3 BUV395 and anti-CD69 BV711 (Panel abbr. CD69). Expression of the CAR has been monitored via intracellular eGFP signal and correlating myc-tag expression on the surface of modified Jurkat cells. The staining with anti-CD3 allowed a separation of CD3+ Jurkat and CD3- target cells during the flow cytometry measurement. Anti-CD69 was utilized to determine the expression level of CD69 after antigen contact. Co-culture assays with nucleofected Jurkat cells were performed twice with duplicates for each condition. To confirm the generated results, the assay was repeated twice as outlined above with transduced Jurkat cells, expressing the vNAR-CAR (1) - (5), with duplicates for each condition. All assays were carried out with SUP-B8 as target cells and SUP-B15 as off-target cells in addition to described *Jurkat only control.* Furthermore, two assays were performed for vNAR-CAR (5)-transduced Jurkats against additional off-targets, such as the IM-9 cell line as well as primary B cells, to confirm the high specificity of the construct in presence of a heterogenous B cell population. Both additional assays were conducted with triplicates per condition. Due to the slow expansion, the CD19-CAR transduced Jurkat cells could not be utilized as a control and were discarded on day 6 after transduction.

### Design and generation of vNAR-CAR constructs

As a proof-of-concept for development of an anti-idiotype B-ALL therapy approach, the present experiments focused on generation and screening of vNAR-CARs targeting the B cell receptor (idiotype) of the B-ALL cell line SUP-B8.

Contrary to non-idiotype therapy approaches, the vNAR-CAR system takes advantage of a selection in favor of anti-idiotype vNARs during yeast surface display, to facilitate a personalized therapy approach.

Exchange of a commonly used scFv towards vNARs as novel binding element and targeting of the SUP-B8 BCR required an adaptation of extracellular spacer domains to provide correct signal transduction and high flexibility. In comparison to common targets for immunotherapy of B-ALL, such as the extracellular domain of CD19, the B cell receptor represents a significantly larger heterotetrametric protein complex on the surface membrane of B cells. Considering size and distance differences of the targeted epitope towards the vNAR-CAR, a screening of various extracellular spacer regions domains was required to select the optimal spacer domain for the vNAR-CAR. Notably, the anti-idiotype vNAR is characterized by a high specificity for idiotype-determining regions inside the antigen-binding fragment (Fab) of the BCR.

Five variants of extracellular vNAR-CAR domains were designed *in silico* utilizing an anti-SUP-B8-BCR vNAR (clone F2). Each construct was designed with a distinct linker length to cover a large distance spectrum for the extracellular spacer domain. For a direct comparison with a previously generated CD19-CAR, the FMC63 scFv was substituted for the vNAR domain, while maintaining the structure of all other CAR elements. This resulted in construct vNAR-CAR (4). The vNAR-CARs (1), (2), (3) and (5) were characterized by relocation of the c-myc-tag to the N-terminus of the vNAR-CAR, for better availability of the protein tag in addition to a shortening of the extracellular domain. Glycine-serine-linkers with one or three G₄S repeats were implemented into vNAR-CAR (2) and (3) respectively. With availability of vNAR-CAR (4), generation of an additional vNAR-CAR with two G₄S repeats was redundant based on the equal amino acid length of the myc-tag. To generate an even shorter extracellular domain for vNAR-CAR (5), the CD8α hinge region was replaced by a minimal glycine-serine-linker with four G₄S repeats.

### Evaluation of vNAR-CAR functionality and efficacy

The next step after optimization of the nucleofection protocol was to conduct a comparative analysis of all generated vNAR-CARs with focus on functionality and specificity. For this work the screening with the modified CD69 T cell activation assay (CD69 assay) utilizes upregulation of CD69 after vNAR-CAR interaction with the B cell receptor (BCR) of target cells as a readout for functional CAR-signal transduction. Specificity of vNAR-CARs for the BCR of SUP-B8 was verified by off-target controls, such as the SUP-B 15 cell line. Initial screenings of vNAR-CAR (1) - (5) were conducted in a nucleofection-based CD69 assay. Subsequently, a lentiviral transduction was performed to achieve a stable long-term expression of vNAR-CARs and an increased viability of Jurkat cells. Transduction-based CD69 assays with all vNAR-CARs were performed, followed by detailed analysis of the lead candidate vNAR-CAR (5) against additional off-targets. It is important to note, that off-target binding of the c-myc PE antibody towards the SUP-B8 target cell line was observed after immunofluorescence staining and following flow cytometry. Due to absence of downstream eGFP in the pSW-CD19-CAR transfer vector, a reliable detection of CD19-CAR+ Jurkat cells via myc-tag and subsequent evaluation of their CD69 expression level could not be accomplished in co-culture with SUP-B8 cells. Therefore, the CD19-CAR construct could not be utilized as a comparative control for vNAR-CARs, and was excluded from data evaluation.

Although the anticipated SUP-B8-specific activation and following upregulation of CD69 was observed without high statistical deviation for vNAR-CAR (3), (4) and (5), only the latter demonstrated superior results with a significant difference (p < 0.0001) against all other conditions. Additionally, vNAR-CAR (4) and (5) were characterized by the lowest off-target effects.

Further evaluation of the calculated vNAR-CAR Signal Intensity/Specificity Score further confirmed the superior specificity and high activation potential of vNAR-CAR (5) with a significant difference (p < 0.0001) against all other vNAR-CARs. Notably, the calculated score of all other vNAR-CAR was considered not significant in comparison to the control, which further highlighted the vNAR-CAR (5) as lead candidate for the further experimental procedure.

The transduction of Jurkat cells with vNAR-CAR (5) resulted in a clear reduction of off-target activation. This was indicated by a minor increase in CD69 expression during normal cultivation and co-culture with the off-target SUP-B 15 cell line, instead of the moderate increase present for nucleofected Jurkat cells. Co-culture of vNAR-CAR (5) Jurkat cells with the target cell line SUP-B8 resulted in a major increase of the CD69 expression and a clear separation of the control- and vNAR-CAR (5) population. As described for nucleofection-based assays, cultivation with SUP-B8 cells led to a minor increase in CD69 expression for control as well as vNAR-CAR (5) Jurkat cells.

In summary, the transduction-based screening provided a more robust method for screening of vNAR-CARs due to lower off-target activation, at expense of a more time-consuming genetic modification of the Jurkat cells. As intended, the PMA/Ionomycin CD69 positive control confirmed the feasibility of the transduction-based CD69 assay.

Determination of the vNAR-CAR Signal Intensity/Specificity Score for vNAR-CAR (5) revealed a significant increase in CD69 upregulation and specificity against the SUP-B8 cell line. In correlation with the decrease in vNAR-CAR surface presentation indicated by a lower signal intensity of the anti-c-myc PE antibody, a corresponding decrease in signal intensity of the vNAR-CAR (5) was observed during conduction of consecutive CD69 assays. Although the magnitude of the activation potential decrease over time, the specificity against the SUP-B8 cell line was confirmed to be significant in all CD69 assays. The detailed evaluation of the acquired data during the CD69 assays on day 15 and 21 revealed a drastic decrease of SUP-B8 cells after co-culture with vNAR-CAR transduced Jurkat cells in comparison to the control.

As mentioned above, activation potential and specificity towards SUP-B8 were evaluated for vNAR-CARs (1)-(5) in nucleofection-based CD69 assays. Compared to the high transfection efficiency achieved during establishment of the nucleofection protocol with the optimized pmaxGFP plasmid (Lonza), nucleofection with the ∼3.5-fold larger pSIEW-vNAR-CAR plasmids resulted in a lower percentage of eGFP+ Jurkat cells. Although pSIEW plasmids can be utilized for nucleofection to achieve transient expression of vNAR-CARs, they were initially designed for application in lentiviral particle production.

Evaluation of the CD69 assay indicated a CD69 upregulation after nucleofection and following co-culture for all conditions. This upregulation can be explained by the combined influence of nucleofection and subsequent gene transfer and was therefore not classified as tonic signaling. Jurkat cells modified with vNAR-CAR (5) were characterized by a high and specific expression of CD69 after coculture with SUP-B8, indicating a functional vNAR-CAR. Additional confirmation was obtained by determination of the vNAR-CAR Signal Intensity/Specificity Score to considered target and off-target specificity and activation potential. The specificity of the shortest construct, vNAR-CAR (5), was significantly superior in comparison to all other vNAR-CARs as tested, which further promotes the thesis, that shortening of the extracellular domains results in an exclusion of signal inhibitory proteins during formation of the immunological synapse.

This in turn maybe explains the higher activation potential demonstrated by vNAR-CAR (5). In conclusion, the nucleofection-based CD69 assays was used as a screening process and enables initial analysis of the generated vNAR-CARs. In addition to CD69 upregulation, the IL-2 production of Jurkat cells after vNAR-CAR specific activation, can be used as a readout.

Further experimental procedure involved production of lentiviral particles for a stable transduction of Jurkat cells with the generated vNAR-CARs. The VSV-G envelope was utilized to promote optimal transduction conditions based on the T cell phenotype of Jurkat cells. Additionally, higher viability and expression level of vNAR-CARs in comparison to the nucleofection-based setting were anticipated. Following the lentiviral transduction of Jurkat cells with vNAR-CAR (1)-(5), cultures were expanded for 31-36 days. The viability, cell count and eGFP/vNAR-CAR expression were monitored during cultivation. Evaluation of viability and cell count revealed a decrease of viable cells and slower proliferation rate during the fourth week of expansion (day 21 to 26). Considering the conducted sub-cultivation in addition to transfer of the cultures into larger cell culture flasks, it is assumed that the cell density was unintendedly adapted to low for the optimal proliferation of the Jurkat cultures. An additional explanation for the slower proliferation and the observed consistent decrease in eGFP and vNAR-CAR expression includes an influence of the viral integration on the proliferation capacity of modified Jurkat cells. Furthermore, selection of the SFFV promotor for a stable long-term expression of vNAR-CARs might be suboptimal, due to epigenetic silencing of the promotor *in vitro* and *in vivo.* In contrast, the human elongation factor 1α (EF1α) promotor represents an excellent alternative to the SFFV promoter, that has been used and tested extensively for immunotherapeutic cell products such as Kymriah.

Although vNAR-CAR (4) demonstrated a high transduction efficacy in regard to the percentage of eGFP+ Jurkat cells, nearly no expression of the myc-tag and corresponding vNAR-CAR was detected. It is assumed, that the myc-tag might not be accessible for the antibody during immunofluorescence staining. Notably, vNAR-CAR (5) revealed superior transduction efficacy and vNAR-CAR expression in comparison to all other vNAR-CARs. Evaluation of the CD69 assays conducted on day 15 and 21 after transduction revealed a highly reduced off-target effect for all vNAR-CARs in comparison to the nucleofection-based CD69 assay. Although each vNAR-CAR indicated a specific activation after co-culture with SUP-B8, as shown by significant upregulation of CD69 in comparison to the control, the signal intensity decreased from day 15 to day 21.

Only vNAR-CAR (5) demonstrated significant results compared to all other vNAR-CARs. As observed during nucleofection-based CD69 assays, vNAR-CAR (5) achieved superior results. Furthermore, the determined signal intensity/specificity score of vNAR-CAR (5) was on a competitive level in comparison to lead candidates described in Bloemberg *et al.* (2020). Subsequent CD69 assays were conducted to verify the specificity of vNAR-CAR (5) against other off-targets, including the cell line IM-9 and a heterogenous population of primary B cells. Analysis of the specificity and activation potential highlighted vNAR-CAR (5) as lead candidate, characterized by the shortest extracellular domain, minimal off-target effects against all off-target cells and specific activation after coculture with SUP-B8. In conclusion, the transduction-based CD69 assay allowed a detailed characterization of vNAR-CAR specificity and was less vulnerable to unintended activation by genetic modification of the Jurkat cells in comparison to the nucleofection-based assay.

### Cytotoxic potential of vNAR-CAR (5)

Preliminary evaluation of a vNAR-CAR-mediated cytotoxic effect on SUP-B8 cells revealed significant decrease of the SUP-B8 population after co-culture with vNAR-CAR (5) modified Jurkat cells. Due to the fact that Jurkat lack significant cytolytic activity, it is assumed that the observed cytotoxic effect is exclusively mediated by the interaction of vNAR and BCR). In this regard, similar effects were achieved by Torchia et al. (2016) (PNAS May 10, 2016 113 (19) 5376-5381) utilizing anti-idiotype peptibodies and Santos et al. (2019) (Braz. J. Pharm. Sci. 54 (spe), 2018, https://doi.org/10.1590/s2175-97902018000001007) using an anti-idiotype monoclonal antibody specific for the BCR of SUP-B8. Hereby, the clustering of the BCR upon interaction with the specific anti-idiotype binding element is proposed to induced apoptosis in B cells. In summary, vNAR-CAR (5) showed not only superior results in comparison to all other generated vNAR-CARs, but furthermore initial cytotoxic effects upon interaction with SUP-B8 cells.

### Literature as cited

Greenberg AS, Avila D, Hughes M, Hughes A, McKinney EC, Flajnik MF. A new antigen receptor gene family that undergoes rearrangement and extensive somatic diversification in sharks. Nature. 1995 Mar 9;374(6518):168-73. doi: 10.1038/374168a0. PMID: 7877689.
Könning, D., et al. (2017). Semi-synthetic vNAR libraries screened against therapeutic antibodies primarily deliver anti-idiotypic binders. Scientific reports, 7(1), 1-13. Könning, D., et al. (2020). Selection and Characterization of Anti-idiotypic Shark Antibody Domains. In Genotype Phenotype Coupling (pp. 191-209). Humana, New York, NY.
Macarrón Palacios A, Grzeschik J, Deweid L, et al. Specific Targeting of Lymphoma Cells Using Semisynthetic Anti-Idiotype Shark Antibodies. Front Immunol. 2020;11:560244. Published 2020 Nov 26. doi:10.3389/fimmu.2020.560244
Müller, S., et al. (2020). High cytotoxic efficiency of lentivirally and alpha retrovirally engineered CD19-specific chimeric antigen receptor natural killer cells against acute lymphoblastic leukemia. Frontiers in immunology, 10, 3123.
Zielonka, S., et al. (2014). Shark attack: high affinity binding proteins derived from shark vNAR domains by stepwise in vitro affinity maturation. Journal of biotechnology, 191, 236-245.
Zielonka S, Empting M, Grzeschik J, Könning D, Barelle CJ, Kolmar H. Structural insights and biomedical potential of IgNAR scaffolds from sharks. MAbs. 2015;7(1):15-25. doi:10.4161/19420862.2015.989032
Granzin, M., et al. (2017). Shaping of natural killer cell antitumor activity by ex vivo cultivation. Frontiers in Immunology, 8, 458.
Bloemberg D, Nguyen T, MacLean S, Zafer A, Gadoury C, Gurnani K, Chattopadhyay A, Ash J, Lippens J, Harcus D, Page M, Fortin A, Pon RA, Gilbert R, Marcil A, Weeratna RD, McComb S. A High-Throughput Method for Characterizing Novel Chimeric Antigen Receptors in Jurkat Cells. Mol Ther Methods Clin Dev. 2020 Jan 31;16:238-254. doi: 10.1016/j.omtm.2020.01.012. PMID: 32083149; PMCID: PMC7021643.

## Claims

1. An scFv chimeric antigen receptor (CAR) molecule comprising at least one variable NAR antigen binding domain, wherein said variable NAR-domain specifically binds to a T cell or B cell specific antigen, and wherein said variable NAR-domain binds to a specific idiotype of a B cell receptor (BCR) or T cell receptor (TCR), and wherein said CAR furthermore comprises at least one extracellular hinge region consisting of a flexible amino acid linker, such as (GGGGS)3-5 or a 6 to 12 glycine linker.

2. The CAR molecule according to claim 1 that is a CAR molecule of the first, second, third, fourth, fifth or next generation, optionally comprising one additional antigen binding domain, such as an scFv antigen binding domain, VHH, DARPIN, or variable NAR-domain.

3. The CAR molecule according to claim 1 or 2, wherein the B or T cell specific antigen is selected from the group consisting of CD1a, CD2, CD3, CD4, CD5, CD13, CD19, CD20, CD22, CD30, CD33, CD34, CD44s, CD52, CD99, CD123, IL7R, CXCR4, survivin, CD138, BCRs, and TCRs.

4. A CAR molecule according to claim 1 or 2, wherein the vNAR is according to SEQ ID NO: 3, or a CAR molecule according to SEQ ID NO: 1, or sequences being at least 95%, preferably at least 97% and most preferably at least 99% identical thereto.

5. A nucleic acid molecule, encoding for the CAR molecule according to any one of claims 1 to 4, wherein said nucleic acid preferably is part of an expression cassette or vector, such as a viral vector.

6. A genetically modified lymphocyte, such as a T cell, NK cell or other immune cell subtype or cell line, expressing the CAR molecule according to any one of claims 1 to 4, wherein preferably said lymphocyte, such as the T cell or NK cell, is derived from an either autologous or third party donor (= allogeneic, haploidentical) origin.

7. A method for producing the CAR molecule according to any one of claims 1 to 4, comprising the steps of
a) identifying at least one anti-idiotype specific variable NAR against a B- or T cell receptor idiotype comprising screening of a naive or semi-synthetic library of potential variable NAR binding domains,
b) including the at least one anti-idiotype specific variable NAR as identified in step a) into a chimeric antigen receptor (CAR) molecule,
wherein said chimeric antigen receptor (CAR) molecule furthermore comprises at least one extracellular hinge region consisting of a flexible amino acid linker, such as (GGGGS)3-5 or a 6 to 12 glycine linker.

8. The method according to claim 7, further comprising the step of humanizing the variable NAR-domain as identified.

9. The method according to claim 7 or 8, wherein the CAR molecule is of the first, second, third, fourth, fifth or next generation, optionally comprising one additional scFv antigen binding domain or variable NAR-domain.

10. The method according to any one of claims 7 to 9, further comprising the step of expressing said CAR molecule in a host cell, such as a vNAR-CAR-T cell or vNAR-CAR-NK cell, preferably in said lymphocyte, such as the T cell or NK cell derived from an either autologous or third party donor (= allogeneic, haploidentical) origin.

11. The method according to claim 10, further comprising the step of detecting the antigen target-specificity, off-target effect, and/or vNAR-CAR-mediated cytotoxicity of said vNAR-CAR-T cell or vNAR-CAR-NK cell against a patient-derived tumor sample, and/or identifying the clonal/antigen-specific idiotype of the B cell receptor (BCR) and/or T cell receptor (TCR) repertoire in a sample of B cells and/or T cells obtained from a patient.

12. A pharmaceutical composition, comprising the CAR molecule according to any one of claims 1 to 4, the genetically modified or recombinant leukocyte, T cell or NK cell according to claim 6, or the vNAR-CAR-T cell or vNAR-CAR-NK cell as produced according to claim 10, together with pharmaceutically acceptable auxiliary agents, preferably a pharmaceutical composition that is suitable for adoptive cell therapy (ACT).

13. The CAR molecule according to any one of claims 1 to 4, the genetically modified or recombinant leukocyte, T cell or NK cell according to claim 6, the vNAR-CAR-T cell or vNAR-CAR-NK cell as produced according to claim 10, or the pharmaceutical composition according to claim 12 for use in the treatment of diseases.

14. The CAR molecule according to any one of claims 1 to 4, the genetically modified or recombinant leukocyte, T cell or NK cell according to claim 6, the vNAR-CAR-T cell or vNAR-CAR-NK cell as produced according to claim 10, or the pharmaceutical composition according to claim 12 for use according to claim 13, wherein said disease is selected from clonal malignancies, such as leukemias, in particular ALL, such as T-ALL and B-ALL.

15. The CAR molecule according to any one of claims 1 to 4, the recombinant leukocyte, T cell or NK cell according to claim 6, the vNAR-CAR-T cell or vNAR-CAR-NK cell as produced according to claim 10, or the pharmaceutical composition according to claim 12 for use according to claim 13, wherein said treatment is personalized, in particular personalized immunotherapy.

16. A method for treating clonal malignancies, such as leukemias, in particular ALL, such as T-ALL or B-ALL, comprising administering to a patient in need of said treatment an effective amount of the CAR molecule according to any one of claims 1 to 4, the genetically modified leukocyte, T cell, NK or other immune cell subtype or cell line according to claim 6, the vNAR-CAR-T cell, vNAR-CAR-NK or vNAR-CAR immune cell as produced according to claim 10, or the pharmaceutical composition according to claim 12, wherein preferably said treatment is personalized, in particular personalized immunotherapy.
